# EUROPÄISCHE PATENTANMELDUNG

(11) **EP 3 738 628 A1**
(43) Veröffentlichungstag der Anmeldung: **18.11.2020**
(21) Anmeldenummer: 19174927.4
(22) Anmeldetag: 16.05.2019
(51) Int. Cl.: A61M 5/32, A61M 5/28, A61M 5/31, A61M 5/315, A61M 5/34

(54) **MEDIZINISCHE SPRITZE MIT PASSIVEM NADELSCHUTZ**

(71) Anmelder: Trenta2 S.r.l., 39100 Bozen (IT)
(72) Erfinder: GALLMETZER, Dietrich, 39018 Terlan (IT)
(74) Vertreter: Tergau & Walkenhorst Patentanwälte PartGmbB

(57) **Zusammenfassung**

Eine Spritze (1) mit passivem Nadelschutz, die mit einer die Retraktion der Spritzennadel (12) auslösenden Sprungfeder (34) versehen ist, soll eine noch weiter verbesserte Zuverlässigkeit und Bedienungssicherheit der Spritze bei Benutzung gewährleisten. Dazu ist erfindungsgemäß vorgesehen, dass die Sprungfeder (34) beim Einziehen der Nadel (12) in den Innenraum des Spritzenkörpers (6) zusätzlich auch die zur Betätigung der Spritze (1) vorgesehene Drückerplatte (20) in eine Richtung weg vom distalen Ende (8) der Spritze (1) verschiebt.

## Beschreibung

Die Erfindung betrifft eine medizinische Spritze mit passivem Nadelschutz.

Zur Vermeidung oder Verringerung von Kontaminations- oder Verletzungsrisiken nach dem Gebrauch von Medikamentenspritzen und insbesondere zur Vermeidung des mehrfachen Gebrauchs von Spritzennadeln durch verschiedene Nutzer finden Spritzen mit so genannten Einzieh- oder Retraktionssystemen für die Spritzennadel zunehmend Verwendung. Bei solchen insbesondere medizinischen Spritzen, auch als "Spitze mit passivem Nadelschutz", wird die Spritzennadel nach Abgabe des in der Spritze vorgehaltenen Wirkstoffs in den Spritzenkörper eingezogen und von diesem vollständig umschlossen. Ein Zugang zur Spritze und damit ein Verletzungsrisiko, oder auch das Risiko mehrfachen Gebrauchs derselben Nadel, kann damit weitgehend ausgeschlossen werden.

Derartige Spritzen mit passivem Nadelschutz sind beispielsweise aus der EP 1 284 769 B1, der EP 0 720 491 B1, der EP 0 680 347 B1 oder der EP 1 764 127 B1 bekannt.

Der Erfindung liegt nunmehr die Aufgabe zugrunde, eine Spritze mit passivem Nadelschutz der oben genannten Art derart weiterzubilden, dass eine noch weiter verbesserte Zuverlässigkeit und Bedienungssicherheit der Spritze bei Benutzung gewährleistet ist.

Diese Aufgabe wird erfindungsgemäß gelöst, indem die Spritze mit einer die Retraktion auslösenden Sprungfeder versehen ist, die beim Einziehen der Nadel in den Innenraum des Spritzenkörpers zusätzlich auch die zur Betätigung der Spritze vorgesehene Drückerplatte in eine Richtung weg vom distalen Ende der Spritze verschiebt.

Die Erfindung geht dabei von der Überlegung aus, dass die angestrebte Einziehung oder Retraktion der Spritzennadel in das Spritzengehäuse hinein dem Benutzer nicht nur optisch, sondern auch auf andere nutzbare Weise, insbesondere mittels haptischer Wahrnehmbarkeit, signalisiert werden sollte. Dazu sollte die Spritze, gekoppelt an den Retraktionsvorgang der Nadelspitze, entsprechend eine Verschiebung ihrer zugeordneten Drückerplatte erzeugen, die für den Benutzer beispielsweise über den Daumen fühlbar ist. Auf diese Weise kann dem Benutzer, ohne dass dieser die Spritze aktiv beobachten müsste, die Information übermittelt werden, dass die Nadel ordnungsgemäß eingezogen wurde, und vor allem auch dass der Wirkstoff vollständig verabreicht wurde und die Wirkstoffabgabe damit beendet ist.

Ein Ausführungsbeispiel der Erfindung wird anhand einer Zeichnung näher erläutert. Darin zeigen:
- Fig. 1: eine medizinische Spritze mit passivem Nadelschutz,
- Fig. 2: eine Patroneneinheit der Spritze nach Fig. 1 in seitlicher Ansicht,
- Fig. 3: die Patroneneinheit nach Fig. 2 im Teilschntt,
- Fig. 4: die Patroneneinheit nach Fig. 2 in Explosionsdarstellung,
- Fig. 5: eine Betätigungseinheit der Spritze nach Fig. 1 in seitlicher Ansicht,
- Fig. 6: die Betätigungseinheit nach Fig. 5 im Teilschnitt,
- Fig. 7: die Betätigungseinheit nach Fig. 5 in Explosionsdarstellung,
- Fig. 8: eine Sequenz von Teilschnitten der Spritze gemäß Fig. 1,
- Fig. 9: eine zur Verwendung in der Spritze gem. Fig. 1 vorgesehene, in einen Nadelhalter eingesteckte Nadel in seitlicher Ansicht,
- Fig. 10: die Nadel gem. Fig. 9 in perspektivischer Ansicht,
- Fig. 11: einen Kolben der Spritze gem. Fig. 1,
- Fig. 12: einen Betätigungsstößel der Spritze gem. Fig. 1,
- Fig. 13: den Betätigungsstößel nach Fig. 12 im Längsschnitt, und
- Fig. 14,15: jeweils eine alternative Ausführungsform einer medizinischen Spritze mit Nadelschutz und Doppelkammersystem im Längsschnitt.

Gleiche Teile sind in allen Figuren mit denselben Bezugszeichen versehen.

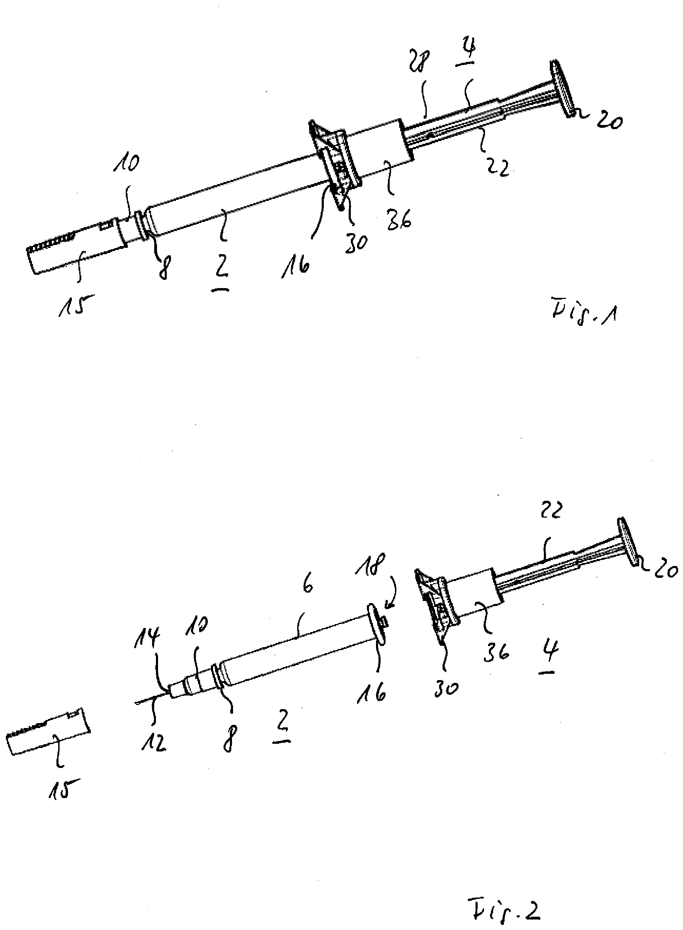

Die medizinische Spritze 1 mit passivem Nadelschutz gemäß Fig. 1 umfasst im Wesentlichen zwei Baugruppen, nämlich einerseits eine Kartuschen- oder Patroneneinheit 2 und andererseits eine Antriebs- oder Betätigungseinheit 4. Im Ausführungsbeispiel ist die medizinische Spritze 1 dabei zweikomponentig aufgebaut, wobei die beiden genannten Baugruppen separate, auf die nachstehend beschriebene Weise miteinander verbindbare Komponenten bilden. Alternativ könnte die Spritze aber auch einkomponentig ausgeführt sein, wobei die beiden genannten Baugruppen von vornherein miteinander verbunden sind und die Unterscheidung in die beiden Baugruppen lediglich funktional erfolgt.

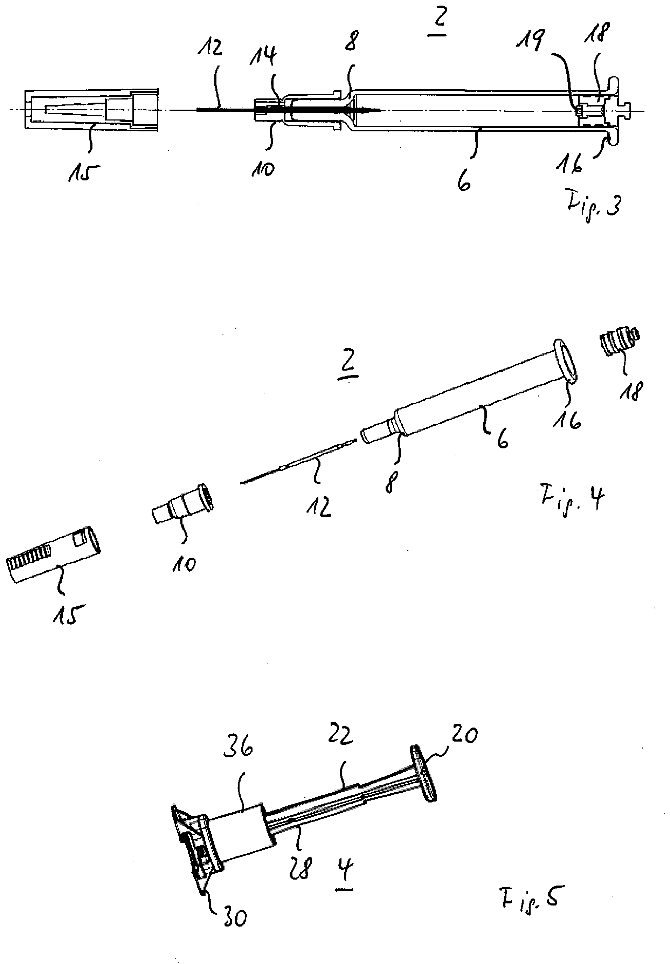

Die in Fig. 2 in einem Zustand vor Verabreichung des medizinischen Wirkstoffs, in Fig. 3 im Teilschnitt und in Fig. 4 in Explosionsdarstellung gezeigte erste Baugruppe, also die Kartuschen- oder Patroneneinheit 2, bildet die eigentliche Spritze und umfasst einen entsprechend einer herkömmlichen Bauweise zylindrisch oder rohrförmig ausgeführten, ein Spritzengehäuse bildenden Hohlkörper 6, in den der medizinische Wirkstoff abgefüllt ist. Am vorderen oder distalen Ende 8 des Hohlkörpers 6 ist ein Nadelhalter 10 befestigt, in dem die zur Injektion des Wirkstoffs vorgesehene Hohlnadel 12 in einer Lagermuffe 14 gelagert ist. Der Nadelhalter 10 könnte dabei einstückig mit dem das Spritzengehäuse bildenden Hohlkörper 6 ausgeführt sein. Im Ausführungsbeispiel ist der Nadelhalter 10 aber in als eigenständig erfinderisch angesehener Ausführung als separates Bauteil ausgestaltet. Der Nadelhalter 10 ist dabei vorliegend auf den das Spritzengehäuse bildenden Hohlkörper 6 bzw. den Spritzenkonus aufgesteckt oder aufsteckbar, könnte aber auch mittels eines Gweindes, beispielsweise eines Luer-Gewindes, anschraubbar ausgeführt sein.

Die Nadel 12 ist zur Vermeidung von Verletzungen oder dergleichen von einer abnehmbaren Schutzkappe 15 umgeben, die vor dem Einsatz der Spritze 1 entfernt wird. Das hintere oder proximale Ende 16 des das Spritzengehäuse bildenden Hohlkörpers 6 ist hingegen von einem in seinen Außenabmessungen passgenau an die Innenkontur des Hohlkörpers 6 angepassten, innerhalb des Hohlkörpers 6 verschiebbaren Kolben 18 verschlossen. Im in Fig. 2 gezeigten Zustand, also vor der Ausbringung des medizinischen Wirkstoffs, ist dieser somit im Inneren des endseitig durch den Kolben 18 verschlossenen Hohlkörpers 6 eingeschlossen. An seiner dem Innenraum des Hohlkörpers 6 zugewandeten Endfläche weist der Kolben 18 mittig ein Aufnahmeloch 19 für die Nadel 12 auf.

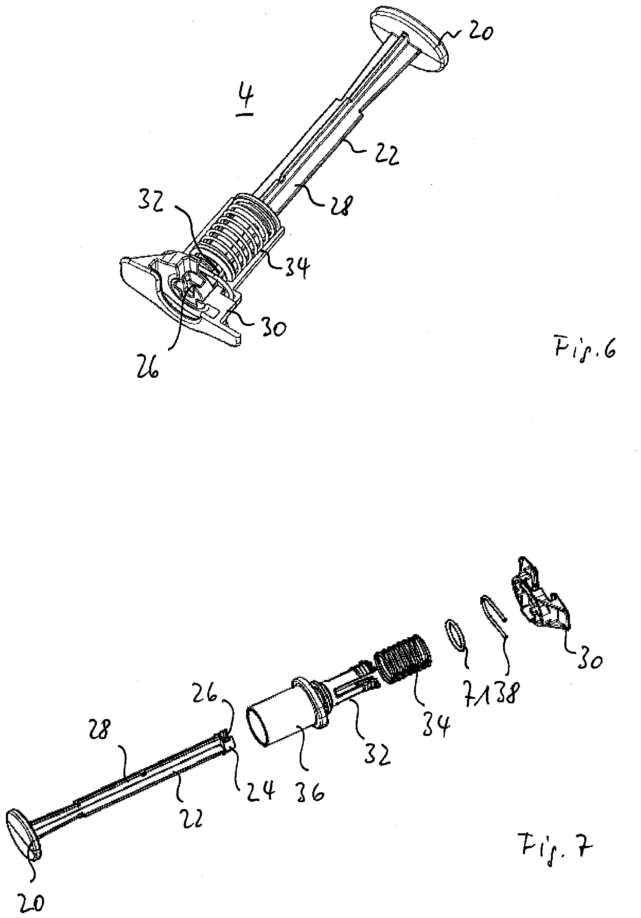

Die in Fig. 5 in seitlicher Ansicht, in Fig. 6 im Teilschnitt und in Fig. 7 in Explosionsdarstellung gezeigte zweite Baugruppe der medizinischen Spritze 1, also die Antriebs- oder Betätigungseinheit 4, umfasst einen endseitig mit einer Drückerplatte 20 versehenen, als längliches Element oder schaftartig ausgestalteten Betätigungsstößel 22. An seinem der Drückerplatte 20 gegenüberliegenden Ende 24 weist der Betätigungsstößel 22 ein zur Verbindung mit dem Kolben 18 vorgesehenes Kuppelelement 26 auf. Mit seinem sich zwischen Drückerplatte 20 und Kuppelelement 26 erstreckenden Schaft 28 ist der Betätigungsstößel 22 durch eine Kuppelplatte 30 hindurch geführt und in dieser in seiner Längsrichtung verschiebbar gelagert. An der von der zur Verbindung mit der Patroneneinheit 2 abgewandten Seite der Kuppelplatte 30 ist ein den Schaft 28 in einem Teilabschnitt umgebendes Arretierelement 32 angeordnet, das seinerseits von einer im in Fig. 6 dargestellten Zustand vor der Verbindung mit der Patroneneinheit 2 unter Vorspannung stehender komprimierter Sprungfeder 34 umgeben ist. Diese Komponenten sind ihrerseits innerhalb eines in der Art eines Röhrenstutzens ausgeführten, an der Kuppelplatte 30 angebrachten Schutzgehäuses 36 positioniert. Im Verbindungsbereich zum Schutzgehäuse 36 ist in der Kuppelplatte 30 zudem eine Sicherungsfeder 38 vorgesehen.

Die medizinische Spritze 1 ist durch die genannten Komponenten und Bauteile mit einem in der Art eines Retraktionssystems ausgeführten passiven Nadelschutz versehen. Damit wird bezweckt, dass nach der Benutzung der Spritze 1, also nach der Ausgabe des im das Spritzengehäuse bildenden Hohlkörper 6 vorgehaltenen Wirkstoffs über die Nadel 12, diese in das Spritzengehäuse derart hineingezogen wird, dass sie vom Spritzengehäuse vollständig umschlossen wird. Damit soll eine unbeabsichtigte Berührung der benutzten Nadel 12, beispielsweise durch Hilfs- oder Pflegepersonal, und somit das Verletzungs- und Kontaminationsrisiko besonders gering gehalten oder möglichst ganz ausgeschlossen werden.

Dafür ist grundsätzlich der folgende Ablauf beim Einsatz und der Handhabung der genannten Komponenten vorgesehen:
Aufgrund der im Ausführungsbeispiel vorgesehenen zweikomponentigen Ausführung des Gesamtsystems werden zunächst die Patroneneinheit 2 und die Betätigungseinheit 4 geeignet miteinander verbunden. Dazu wird einerseits das Kuppelelement 26 mit dem Kolben 18 und andererseits die Kupplungsplatte 30 mit dem proximalen Ende 16 des Hohlkörpers 6 verbunden. Bei von vornherein einkomponentiger Ausführung des Gesamtsystems, bei dem beispielsweise der Betätigungsstößel 22 unmittelbar mit dem Kolben 18 verbunden und die Kupplungsplatte 30 unmittelbar an das proximale Ende 16 des Hohlkörpers 6 angeformt sein könnte, kann dieser Verbindungsschritt naturgemäß entfallen.

Nachdem die Spritze 1 auf diese Weise einsatzfertig gemacht wurde, wird die Nadel 12 zur Verabreichung des medizinischen Wirkstoffs geeignet am Patienten positioniert, so dass sie an geeigneter Stelle die Haut des Patienten durchstößt. Die Haltekreft der Nadel 12 in der Lagermuffe 14 ist dabei, insbesondere durch geeignete Dimensionierung der Komponenten und/oder die Wahl der Materialpaarung, derart vorgegeben, dass die Nadel 12 sicher in ihrer Position in der Lagermuffe 14 verbleibt, wenn der Bediener durch Handhabung am Hohlkörper 6 die Nadel 12 durch die Haut des Patienten hindurchsticht.

Anschließend wird der Betätigungsstößel 22 vom Bediener gedrückt, so dass sich der Kolben 18 innerhalb des Hohlkörpers 6 auf dessen distales Ende 8 hinzu bewegt und dabei den medizinischen Wirkstoff der Nadel 12 zuführt und über diese ausbringt. Kurz vor vollständiger Ausbringung des Wirkstoffs, also kurz vor vollständiger Entleerung des Innenraums des Hohlkörpers 6, erreicht der Kolben 18 in der Nähe des distalen Endes 8 des Hohlkörpers 6 das in den Innenraum ragende Ende der Nadel 12, so dass diese bei weiterer Bewegung in das dafür vorgesehene Aufnahmeloch 19 eindringt. Nach vollständiger Ausbringung des Wirkstoffs erreicht der Kolben 18 dann seine Endposition unmittelbar am distalen Ende 8 des Hohlkörpers 6 und umschließt dabei den in das Aufnahmeloch 19 hineinragenden Teil der Hohlnadel 12. Diese Einbringung des entsprechenden Teilbereichs der Hohlnadel 12 in das Aufnahmeloch 19 und die dadurch erreichte Verbindung der Nadel 12 mit dem Kolben 18 wird vorliegend auch als "connecting" bezeichnet.

Sobald dieser Punkt erreicht und der Kolben 18 an seiner vorgesehenen Endposition angelangt ist, soll sich das Arretierelement 32 - im Ausführungsbeispiel über eine Einrastung am Betätigungsstößel 22 - geeignet verformen und dabei die Fixierung der Sprungfeder 34 lösen. Im Ausführungsbeispiel ist die Dimensionierung der entsprechenden Komponenten dabei derart gewählt, dass das "conecting" etwas oder kurzzeitig vor der Auslösung der Sprungfeder 34 erfolgt; dies wird als eigenständig erfinderisch angesehen. In Reaktion darauf, also auf die Auslösung der Sprungfeder 34, entspannt sich die vorgespannte Sprungfeder 34 und bewegt dabei die Drückerplatte 20 des Betätigungsstößels 22 von der Kupplungsplatte 30 und somit vom durch den Hohlkörper 6 gebildeten Spritzenkörper weg. Damit wird auch der über das Kuppelelement 26 mit dem Schaft 28 des Betätigunsstößels 22 verbundene Kolben 18 mitgenommen und innerhalb des Hohlkörpers 6 vom distalen Ende 8 weg zum proximalen Ende 16 hin gezogen. Dabei nimmt er seinerseits die eingefasste Nadel 12 mit und zieht diese in den Hohlkörper 6 hinein, so dass diese im Endzustand vollständig innerhalb des Hohlkörpers 6 positioniert ist.

Ein bedeutsamer und als erfinderisch angesehener Aspekt ist hierbei, dass die zur Durchführung der Retraktionsbewegung der Nadel 12 in den Hohlkörper 6 hinein vorgesehene Sprungfeder 34 oberhalb der Kupplungsplatte 30 im Bereich des Schafts 28 des Betätigungsstößels 22 angeordnet ist und über dessen Dückerplatte 20 auf den Betätigungsstößel 22 wirkt. Im Gegensatz zu üblichen Retraktionssystemen, bei denen die Retraktionsfeder im Bereich der Spitze oder des distalen Endes der Spritze innerhalb des Spritzenkörpers angeordnet ist und direkt auf die Nadel oder deren Lagerkörper einwirkt, bedeutet die nunmehr erfindungsgemäß vorgesehene Auslegung, dass die Nadel 12 bei der Retraktion gemeinsam mit dem Betätigungsstößel 22 und insbesondere dessen Drückerplatte 20 bewegt wird, so dass der Benutzer oder Bediener anhand der Position der Drückerplatte 20 relativ zur Kupplungsplatte 30 die Position und vor allem die Positionsänderung der Nadel 12 erkennen kann. Diese Erkennung ist optisch möglich, vor allem aber auch haptisch.

Mit anderen Worten erzeugt die Retraktionsbewegung, ähnlich wie bei einem Betätigungsknopf eines Kugelschreibers, eine für den Benutzer beispielsweise mit dem Daumen spürbare Bewegung der Drückerplatte 20. Damit kann der Benutzer aus der mit dem Daumen spürbaren Bewegung der Drückerplatte 20 einerseits darauf schließen, dass die Nadel 12 nunmehr eingezogen ist und somit im Bereich des distalen Endes 8 der Spritze 1 keine Verletzungs- oder Kontaminationsgefahr mehr besteht. Andererseits kann er daraus aber auch schließen, dass der Wirkstoff nunmehr vollständig verabreicht wurde, ohne dies beispielsweise optisch überwachen zu müssen. Die Spritze 1 ermöglicht somit eine deutlich vereinfachte Handhabung bei der Verabreichung des Wirkstoffs.

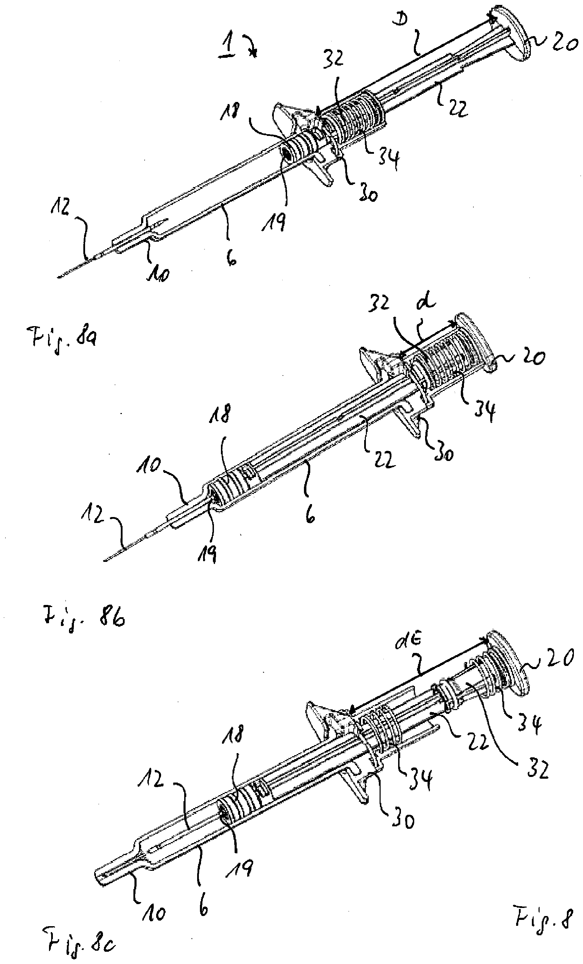

Zur besseren Verdeutlichung ist diese Abfolge in der Sequenz von Teilschnitten in Fig. 8 dargestellt. Darin zeigt Fig. 8a die Spritze 1 vor Verabreichung des im innenruam des Hohlkörpers 6 vorgehaltenen Wirkstoffs. Der im Innenraum des Hohlkörpers 6 geführte Kolben 18 befindet sich dabei an seinem Anschlag an der am proximalen Ende 16 des Hohlkörpers 6 angeordneten Kupplungsplatte 30. Der Betätigungsstößel 22 ist dementsprechend voll ausgefahren, und die Drückerplatte 20 befindet sich im maximalen Abstand D von der Kupplungsplatte 30. Die Sprungfeder 34 ist über das Arretierelement 32 vorgespannt gehalten und im unmittelbar der Kupplungsplatte 30 benachbarten Teilabschnitt des Befestigungsstößels 22 positioniert. Die im Nadelhalter 10 gehaltene Hohlnadel 12 ist in diesem Zustand einsatzbereit ausgefahren.

Ausgehend von diesem Zustand wird - insbesondere bei in die Haut des Patienten eingestochener Nadel 12 - der Wirkstoff ausgebracht, indem der Kolben 18 von seiner in Fig. 8a gezeigten Ausgangsposition bis zu der in Fig. 8b gezeigten Endposition unmittelbar benachbart zum distalen Ende 8 des Hohlkörpers 6 hin verschoben wird. Zu diesem Zweck wird der Betätigungsstößel 22 in den Hohlkörper 6 hineingeschoben bis hin zur Endposition, in der die Drückerplatte 20 den minimalen Abstand d von der Kupplungsplatte 30 einimmt. In diesem momentanen Zustand ist die Sprungfeder 34 zwar immer noch vorgespannt, aber durch die vorgegebene Form des Betätigungsstößels 22 wird das Arretierelement 32 beim Erreichen dieser Position ausgelöst. Die im Nadelhalter 10 gehaltene Hohlnadel 12 ist in diesem Zustand immer noch ausgefahren, ist aber bereits in die Aufnahmeöffnung 19 des Kolbens 18 eingedrungen und wird mit ihrem in den Innenraum des Hohlkörpers 6 ragenden Ende vom Kolben 18 umfasst und mit diesem mechanisch verbunden.

Bei Erreichen der in Fig. 8c gezeigten Endposition des Kolbens 18 bzw. des Betätigungsstößels 22 wird das Arretierelement 32 ausgelöst, so dass es die vorgespannte Sprungfeder 34 freigibt. In Reaktion darauf dehnt sich die Sprungfeder 34 in ihrer Längsrichtung aus, und da sie rückseitig an der Kupplungsplatte 30 abgestützt ist, übt sie eine entsprechende Rückstellkraft auf die Drückerplatte 20 aus und verschiebt diese in die Richtung von der Kupplungsplatte 30 weg, bis zur Endposition, die in Fig. 8c gezeigt ist. In dieser Endposition ist die Sprungfeder 34 entspannt, und die Drückerplatte 20 befindet sich in einer Position mit dem Endabstand dE von der Kupplungsplatte 30. Entsprechend hat sich der Betätigungsstößel 22 dabei mitbewegt, so dass sich in dieser Position der Kolben 18 in einer mittleren Position innerhalb des Hohlkörpers 6 befindet. Die Hohlnadel 12 wurde dabei vom Kolben 18 mitgenommen und befindet sich somit nunmehr vollständig innerhalb des Hohlkörpers 6.

Wie den Darstellungen in Fig. 8 entnehmbar ist, ist der Endabstand dE kleiner als der ursprüngliche, maximale Abstand D, aber größer als der zwischenzeitlich eingenommene minimale Abstand d. Dies bedeutet, dass die Drückerplatte 20 nach vollständiger Ausbringung des Wirkstoffs selbsttätig "ausfährt" und sich nach oben, weg von der Drückerplatte 20, bewegt. Damit erhält der Benutzer ein haptisches, beispielsweise über den Daumen fühlbares Feedback-Signal, dass der Wirkstoff vollständig ausgebracht wurde und sich die Nadel nunmehr gesichert im Innenraum des Hohlkörpers befindet.

Um die oben erläuterte Funktionsweise auf besonders zuverlässige und in mehrfacher Hinsicht vorteilhafte Weise zu erreichen, sind die Komponenten in diversen Details spezifisch ausgestaltet, wobei die nachfolgend beschriebenen Ausgetaltungen jeweils sowohl als eigenständig erfinderisch als auch in beliebiger Kombination miteinander als erfinderisch angesehen werden.

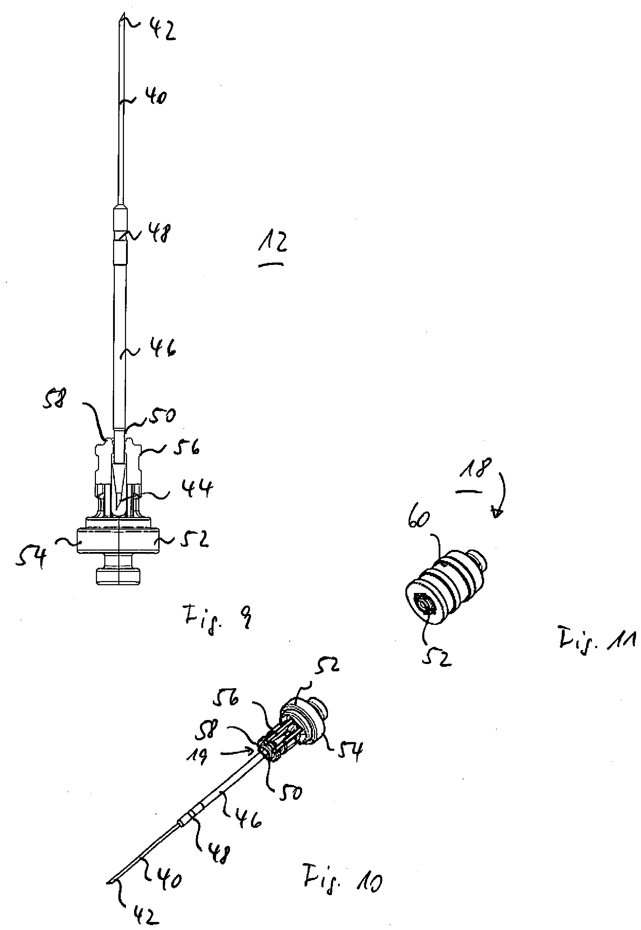

So ist beispielsweise die Hohlnadel 12 gemäß nachfolgender Beschreibung eigenständig erfinderisch ausgeführt. Wie der vergrößerten Darstellung in den Fig. 9 (seitliche Ansicht) und Fig. 10 (perspektivische Ansicht) entnehmbar ist, umfasst die Hohlnadel 12 in eigenständig erfinderischer Ausgestaltung ein Nadelrohr 40 aus Metall, das an seinen beiden Enden jeweils eine Nadelspitze 42, 44 bildet. Die Materialwahl für das Nadelrohr 40 ist dabei bevorzugt im Hinblick auf gängige Erfordernisse für medizinische Anwendungen geeignet erfolgt, wobei besonders bevorzugt ein auch für Standard-Nadeln verwendbares rostfreies Material vorgesehen ist. In seinem mittteleren Längenbereich ist das Nadelrohr 40 ummantelt ausgeführt und von einem Kunststoffmantel 46 umgeben. Als Material für den Kunststoffmantel 46 ist dabei vorzugsweise ein Polyamid (PA12), ganz besonders bevorzugt das im Handel unter der Bezeichnung Vestamid Care ML 17 erhältliche, vorgesehen. Der Kunststoffmantel 46 wird dabei in einer ganz besonders bevorzugten, ebenfalls als eigenständig erfinderisch angesehenen Ausgestaltung auf das Nadelrohr 40 aufgespritzt, nachdem dieses in der Art einer Oberflächenaktivierung einer Plasmavorbehandlung unterzogen wurde. Damit ist eine besonders gute Haftung des den Kunststoffmantel 46 bildenden Kunststoffs auf der Nadel 40 erreichbar. Im Kunststoffmantel 46 sind zwei Haltenuten 48, 50 ausgeformt, die die oben beschriebene Funktionsweise ermöglichen sollen.

Die erste Haltenut 48 ist dabei für die vorübergehende Fixierung der Nadel 12 in der Lagermuffe 14 des Nadelhalters 10 vorgesehen. Dazu ist innerhalb der Lagermuffe 14 eine zugeordnete umlaufende Rastlippe vorgesehen, die bei montierter und in die Lagermuffe 14 ordnungsgemäß eingeschobener Nadel 12 in die Haltenut 48 eingreift und diese in Längsrichtung fixiert. Gemäß einer grundsätzlich als eigenständig erfinderisch angesehenen Ausgestaltung sind die Dimensionierungen der Haltenut 48 und der Rastrille dabei vorteilhafterweise derart gewählt, dass unter Berücksichtigung der Verformbarkeit des Materials des Kunststoffmantels 46 und/oder einer eventuellen Klebekraft durch die Materialpaarung des Materials des Kunststoffmantels 46 und des diesen umgebenden Materials der Lagermuffe 14 die Halte- oder Losbrechkraft der solchermaßen eingerasteten Nadel 12 in Längsrichtung einerseits ausreichend groß ist, so dass die Nadel 12 entsprechend der oben beschriebenen Vorgehensweise in die Haut des Patienten eingestochen werden kann, andererseits aber auch ausreichend klein ist, so dass die beschriebene Retraktionsbewegung der Nadel 12 zum Inneren des Hohlkörpers 6 hin durchgeführt werden kann. Gegebenenfalls kann das Profil der Haltenut 48 auch entsprechend asymmetrisch ausgeführt sein, mit einem vergleichsweise steilen Flankenwinkel an ihrer der dem Innenraum zugewandten Spitze 44 zugewandten Seite und einem vergleichsweise flachen Flankenwinkel an ihrer der freiliegenden Spitze 42 zugewandten Seite.

Die zweite Haltenut 50 ist hingegen für eine entsprechende Verrastung im Kolben 18 vorgesehen. In den Figs. 9, 10 ist die Nadel 12 im in den Kolben 18 eingesteckten Zustand gezeigt. Der Kolben 18 ist dabei seinerseits mehrteilig ausgeführt und umfasst den in den Figs. 9, 10 ebenfalls gezeigten Nadelhalter 52, der seinerseits als eigenständig erfinderisch angesehen wird. Der Nadelhalter 52 ist dabei als Kunststoffteil ausgeführt und besteht im Ausführungsbeispiel im Hinblick auf die erforderllichen Haltekräfte und die bei bestimmungsgemäßem Gebrauch erwarteten mechanischen Belastungen, aber auch im Hinblick auf zulassungsbedingte Erfordernisse aus dem unter der Bezeichnung "Bormed™" (HD810MO, ISO 10993 Information (Biocompatibility)) erhältlichen Polypropylen.

Wie der Darstellung in Fig. 9, besonders gut aber auch der perspektivischen Ansicht in Fig. 10 entnehmbar ist, umfasst der Nadelhalter 52 einen an einen Grundkörper 54 angeformten Haltebügel 56, der das eigentliche, das Aufnahmeloch 19 bildende Nadellager 58 trägt. Das Nadellager 58 ist dabei, analog der oben beschriebenen Lagermuffe 14, innenseitig mit einer zugeordneten umlaufenden Rastlippe versehen, die bei in das Nadellager 58 eingeschobener Nadel 12 in die zweite Haltenut 50 eingreift und diese in Längsrichtung fixiert. Gemäß einer grundsätzlich ebenfalls als eigenständig erfinderisch angesehenen Ausgestaltung sind die Dimensionierungen der Haltenut 50 und der dieser zugeordneten Rastrille im Nadellager 58 dabei vorteilhafterweise derart gewählt, dass unter Berücksichtigung der Verformbarkeit des Materials des Kunststoffmantels 46 und/oder einer eventuellen Klebekraft durch die Materialpaarung des Materials des Kunststoffmantels 46 und des diesen umgebenden Materials des Nadellagers 58 die Halte- oder Losbrechkraft der solchermaßen eingerasteten Nadel 12 in Längsrichtung größer ist als die enstprechende Halte- oder Losbrechkraft der Haltenut 48 in der Lagermuffe 14, so dass bei der Retraktionsbewegung des Kolbens 18 die Nadel 12 von diesem zum Inneren des Hohlkörpers 6 mitgenommen wird.

Durch die Ausgestaltung des Haltebügels 56 wird zudem innerhalb des Kolbens 18 ein Freiraum geschaffen, der in der Endphase der Ausbringung des Wirkstoffs, bei der die Nadelspitze 44 schon in das Aufnahmeloch 19 eingedrungen und somit für den Wirkstoff nicht mehr ohne weiteres zugänglich ist, in der Art eines Bypasses das Einströmen des Wirkstoffs über die Nadelspitze 44 in das Nadelrohr 40 ermöglicht. Der Zustrom kann dabei beidseitig des Haltebügels 56 in den Freiraum hinein erfolgen.

Wie bereits ausgeführt, ist in einer weiteren, ebenfalls als eigenständig erfinderisch angesehenen Ausgestaltung der Kolben 18 mehrteilig ausgeführt. Wie der vergrößerten Ansicht gem. Fig. 11 entnehmbar ist, ist dabei zusätzlich zu dem bereits beschriebenen Nadelhalter 52 für die Nadel 12 ein diesen umgebender Kolbenmantel 60 vorgesehen. Der Kolbenmantel 60 besteht dabei unter Berücksichtigung zulassungsbedingter Erfordernisse aus herkömmlichem Gummi, besonders bevorzugt aus dem von der Firma Kreiburg unter der Bezeichnung TM4RST (MC/RS Series) erhältlichen Material. Der Kolbenmantel 60 ist dabei derart geformt, dass er bei eingebrachtem Nadelhalter 52 beidseitig des Haltebügels 56 Zuströmkanäle für den Wirkstoff freilässt, so dass im Sinne einer Vermeidung oder Minimierung des Totvolumens der Bypass für den angestrebten Nullvolumen-Ausstoß gebildet wird.

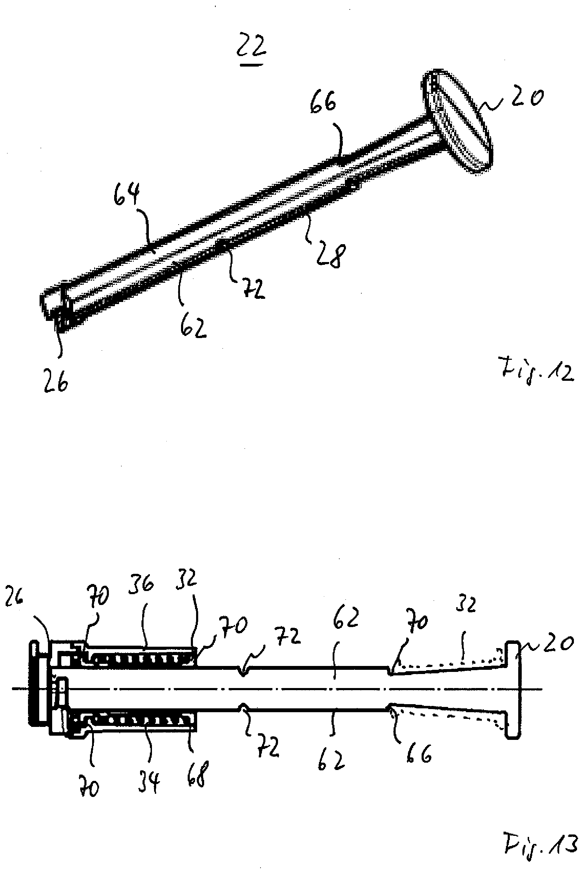

Um die vorgesehene Auslösung des Arretierelements 32 zur Freigabe der vorgespannten Sprungfeder 34 zu ermöglichen, ist eine spezifische, ebenfalls als eigenständig erfinderisch angesehene Ausgestaltung des Schafts 28 des Betätigungsstößels 22 vorgesehen. Zur Verdeutlichung ist dieser vergrößert in Fig. 12 dargestellt. Wie dieser Darstellung entnehmbar ist, wird der Schaft 28 im Wesentlichen durch zwei gekreuzte, im Querschnitt ein Kreuz bildende Schaftrippen 62, 64 gebildet. Wie der Darstellung im Längsschnitt gem. Fig. 13 entnehmbar ist, verlaufen die Oberkanten der Schaftrippen 62, 64 in Längsrichtung des Betätigungsstößels 22 gesehen im Wesentlichen geradlinig und über weite Teile der Gesamtlänge des Betätigungsstößels 22 im Wesentlichen parallel zu dessen Längsrichtung. An einer Position relativ nahe zur Drückerplatte 20 weisen die Schaftrippen 62, 64 jedoch einen Rücksprung 66 auf, so dass sich hier eine Kante bildet.

Im einsatzbereiten Zustand der Spritze 1 ist dabei, wie in Fig. 13 links dargestellt, das Arretierelement 32, das einige vorgespannte Schenkel 68 bildet, im endseitigen Bereich auf den Schaft 28 aufgeschoben. Die Schaftrippen 62, 64 stützen dabei die vorgespannten Schenkel 68 ab, so dass diese gerade ausgerichtet bleiben. Endseitig ist an den Schenkeln 68 eine Stützwulst 70 angeformt, auf der die außenseitig auf dem Arretierelement 32 vorgesehene vorgespannte Sprungfeder 34 abgestützt ist. Durch die Abstützung wird die Vorspannung der Sprungfeder 34 aufrechterhalten.

Falls nun, entsprechend dem in Fig. 8b dargestellten Zustand der Spritze 1, bei der Verabreichung des Wirkstoffs die Endposition des Kolbens 18 im Hohlkörper 6 erreicht wird, überstreicht das Arretierelement 32 in seiner gesamten Länge den Rücksprung 66 des Schafts 28, so dass die Abstützung der vorgespannten Schenkel 68 nach innen hin wegfällt. Dadurch bedingt weichen die Schenkel 68 getrieben durch ihre Vorspannung nach innen hin weg, wie dies in strichlierter Form in Fig. 13 rechts angedeutet ist. Damit weichen auch die Stützwulste 70 nach innen hin weg, so dass die Abstützung der Sprungfeder 34 entfällt und diese sich entspannen kann. Damit wird die gewünschte Retraktion in Gang gesetzt.

Des Weiteren ist in den Schaftrippen 62, 64 noch eine Kerbe 72 vorgesehen, in die bei entsprechender Positionierung die in der Kuppelplatte 30 angeordnete Sicherungsfeder 38 einrasten kann. Dies dient dazu, die eingenommene Position nach erfolgter Retraktion der Nadel 12 in das Innere des Hohlkörpers 6 hinein zu fixieren, so dass die Nadel 12 dort gesichert verbleibt.

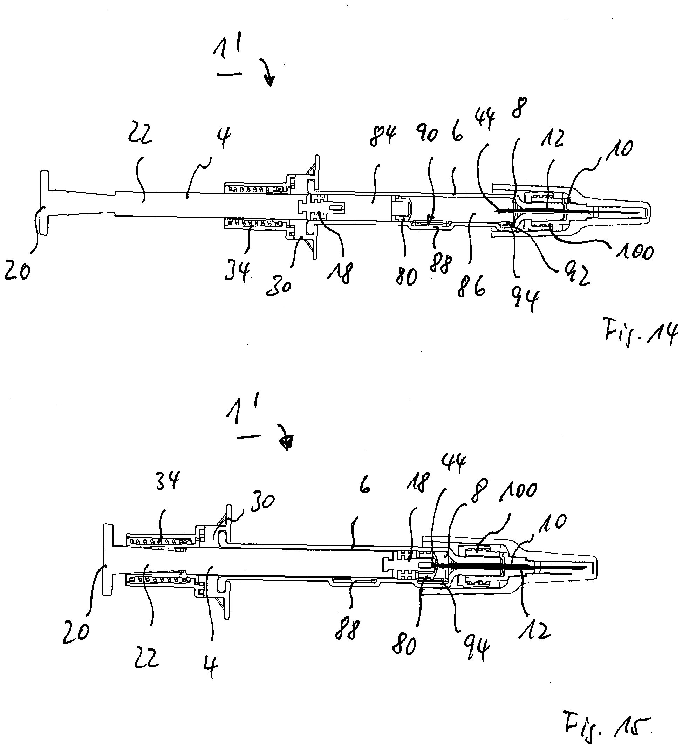

Eine alternative, ebenfalls als eigenständig erfinderisch angesehene Ausführungsform einer medizinischen Spritze 1' mit in der Art eines Retraktionssystems ausgeführtem passiven Nadelschutz der genannten Art ist in den Figs. 14, 15 im Längsschnitt gezeigt. Fig. 14 zeigt die medizinische Spritze 1' dabei vor, Fig. 15 nach der Ausbringung des Wirkstoffs kurz vor Auslösung des Retraktionsmechanismus und Einziehen der Nadel 12. In dieser alternativen Ausführungsform ist die medizinische Spritze 1' mit einem Doppelkammer- oder Doppelkolbensystem ausgestattet. In einer ansonsten zur vorgenannten Ausführungsform weitgehend gleichen Bauweise weist die medizinische Spritze 1' dabei zusätzlich zum Kolben 18 innerhalb des Hohlkörpers 6 noch einen weiteren, vorgelagerten Kolben 80 auf. Durch den Zwischenraum zwischen dem Kolben 18 und dem weiteren Kolben 80 wird dabei eine erste Wirkstoffkammer 84 gebildet, und zwischen dem weiteren Kolben 80 und dem apikalen Ende 8 des Hohlkörpers 6 befindet sich dann die zweite Wirkstoffkammer 86.

Ein solches Doppelkammersystem wird insbesondere eingesetzt für flüssige und/oder lyophilisierte (gefriergetrocknete) oder pulverförmig vorliegende Medikamente, die vor der Verabreichung gelöst werden müssen. Solche Doppelkammer-Systeme sind somit eine besonders geeignete Lösung für lyophilisiert /flüssig oder flüssig/flüssig Wirkstoffkombinationen. Das System bietet eine Vielzahl von Vorteilen für empfindliche Injektionsmedikamente.

In der ersten Wirkstoffkammer 84 wird dabei beispielsweise das Lösungsmittel vorgehalten, wohingegen sich der eigentliche, z. B. gefriergetrocknete oder pulverförmige Wirkstoff in der zweiten Wirkstoffkammer 86 befindet. Bei der Verabreichung des Wirkstoffs wird durch Betätigung des Betätigungsstößels 4 dabei zunächst das in der ersten Wirkstoffkammer 84 bereitgestellte Lösungsmittel über einen in der Gehäusewand des Hohlkörpers 6 angeordneten, von einer Ausformung 88 im Gehäusemantel gebildeten Bypasskanal 90 am weiteren Kolben 80 vorbei in die zweite Wirkstoffkammer 86 eingebracht. Dort löst es den dort vorgehaltenen Wirkstoff, so dass dieser verabreichungsfertig ist. Anschließend wird durch weitere Betätigung des Betätigungsstößels 4 der Kolben 18 bis zum Anschlag an den Kolben 80 bewegt, und anschließend werden die Kolben 18, 80 gemeinsam weiterbewegt, so dass der in der zweiten Wirkstoffkammer 86 befindliche, nummehr gelöste Wirkstoff über die Nadel 12 ausgebracht wird.

Sobald der weitere Kolben 80 die Nadel 12 erreicht und diese in den Kolbenkörper eindringt, wird der restliche Wirkstoff ausgebracht und anschließend, nach der in Fig. 15 gezeigten Position, das Retraktionssystem gemäß der vorstehend beschriebenen Funktionsweise ausgelöst. Die Verringerung des Totvolumens im Endbereich des Hohlkörpers 6 wird dabei durch eine weitere, einen Bypasskanal 92 bildende Ausformung 94 ermöglicht, über die die Restmenge an Wirkstoff vergleichbar zur oben bereits beschriebenen Ausführung dem Ende 44 der Nadel 12 zuströmen kann.

Auch in dieser alternativen Ausführunsgform der medizinischen Spritze 1' könnte der Nadelhalter 10 einstückig mit dem das Spritzengehäuse bildenden Hohlkörper 6 ausgeführt sein. Im vorliegend dargestellten, als eigenständig erfinderisch angesehenen Ausführungsbeispiel ist der Nadelhalter 10 aber als separates Bauteil ausgestaltet und mittels eines Gewindes, vorliegend insbesondere eines Luer-Gewindes 100, an den Hohlkörper 6 anschraubbar ausgeführt. Alternativ könnte der Nadelhalter 10 auch auf den das Spritzengehäuse bildenden Hohlkörper 6 bzw. den Spritzenkonus aufgesteckt oder aufsteckbar ausgeführt sein.

### Bezugszeichenliste

- 1, 1': Medizinische Spritze
- 2: Kartuschen- oder Patroneneinheit
- 4: Antriebs- oder Betätigungseinheit
- 6: Hohlkörper
- 8: distales Ende
- 10: Nadelhalter
- 12: Hohlnadel
- 14: Lagermuffe
- 15: Schutzkappe
- 16: proximales Ende
- 18: Kolben
- 19: Aufnahmeloch
- 20: Drückerplatte
- 22: Betätigungsstößel
- 24: Ende
- 26: Kuppelelement
- 28: Schaft
- 30: Kuppelplatte
- 32: Arretierelement
- 34: Sprungfeder
- 36: Schutzgehäuse
- 38: Sicherungsfeder
- 40: Nadelrohr
- 42,44: Nadelspitze
- 46: Kunststoffmantel
- 48,50: Haltenut
- 52: Nadelhalter
- 54: Grundkörper
- 56: Haltebügel
- 58: Nadellager
- 60: Kolbenmantel
- 62, 64: Schaftrippe
- 66: Rücksprung
- 68: Schenkel
- 70: Stützwulst
- 72: Kerbe
- 80: Kolben
- 84, 86: Wirkstoffkammer
- 88,94: Ausformung
- 90,92: Bypasskanal
- 100: Luer-Gewinde

- D: maximaler Abstand
- d: minimaler Abstand
- dE: Endabstand

## Patentansprüche

1. Spritze (1) mit passivem Nadelschutz, die mit einer die Retraktion der Spritzennadel (12) auslösenden Sprungfeder (34) versehen ist, wobei die Sprungfeder (34) beim Einziehen der Nadel (12) in den Innenraum des Spritzenkörpers (6) zusätzlich auch die zur Betätigung der Spritze (1) vorgesehene Drückerplatte (20) in eine Richtung weg vom distalen Ende (8) der Spritze (1) verschiebt.
